# EUROPEAN PATENT SPECIFICATION

(11) **EP 2 095 841 B1**
(45) Date of publication and mention of the grant of the patent: **07.11.2012**
(21) Application number: 08003777.3
(22) Date of filing: 29.02.2008
(51) Int. Cl.: A61M 39/10, A61M 39/14

(54) **Connection system for connecting a fluid line to a fluid reservoir**
Anschlusssystem zum Anschließen einer Flüssigkeitsleitung an einen Flüssigkeitsbehälter
Système de connexion pour connecter une ligne fluide sur un réservoir fluide

(43) Date of publication of application: 02.09.2009
(73) Proprietor: F.Hoffmann-La Roche AG, 4070 Basel (CH); Roche Diagnostics GmbH, 68305 Mannheim (DE)
(72) Inventor: Scheurer, Simon, 3006 Bern (CH)
(74) Representative: Rentsch Partner AG

(56) References cited:
- WO-A-91/05581
- WO-A-93/10846
- WO-A-2006/077262
- WO-A-2006/121921
- DE-U1- 20 302 788
- FR-A- 2 685 209
- FR-A- 2 826 871
- US-A- 5 122 123
- US-A1- 2002 173 769
- US-A1- 2003 060 804

## Description

The invention relates to a connection system for connecting a fluid line to a fluid reservoir located within a device, in particular within a medical device such as an insulin pump.

It is known to connect a fluid line to a fluid reservoir by way of adapted connectors. The reservoir may be an ampoule provided with a connector to which a so called Luer connector fixed to one end of the fluid line can be attached. Such connection systems are for example used in medical pumping devices such as portable insulin pumps wherein the ampoule contains insulin and is placed within the pump. The insulin is dosed by the pump and fed via the fluid line to the patient in a known manner. US-A-5 122 123 shows a fluid line connector with a hollow needle mounted in a female receiving connector that receives a male connector. The hollow needle is protected against microorganisms by a rubber septum fixed to a collapsible tube. FR-A-2 826 871 shows a fluid line connector as well wherein a hollow needle is mounted in a female receiving connector. US-A 5 492 147 shows a dry break coupling for connecting two fluid lines.

It is the object of the present invention to provide an improved connection system.

This object is met by a connection system for connecting a fluid line to a fluid reservoir located within a device by way of a cannula, comprising
- a cannula holder holding the cannula and comprising a movable cannula cover and locking means for said cannula cover, preventing the movement of the cannula cover by locking said cannula cover in a first state covering the cannula,
- a receiving element for receiving the cannula holder, the receiving element being adapted for fixation to the device or the receiving element forming part of the device, said receiving element comprising releasing means adapted for interaction with the locking means, and wherein
- the releasing means and the locking means are arranged to release the cannula cover by said releasing means interacting by arranged with the locking means, so that the cannula cover is movable by the inserting force to expose the cannula when the cannula holder is inserted into the receiving element, so that the cannula cover is in a second state exposing the cannula when the cannula holder is inserted within the receiving element.

By this connection system the cannula is protected automatically, giving on the one hand secure operation to the operator preventing injury risks by the cannula and on the other hand preventing staining of the cannula. The connection system can be easily adapted for one-hand operation.

In a preferred embodiment of the connection system the cannula cover is moved by a energy storage means, and in particular a coil spring, in opposite direction compared to the movement during insertion, while the cannula holder is removed from the receiving element. This helps considerably for a one-hand operation if the energy storage element is selected such that it actively pushes the cannula holder out of the receiving element when the connection is released again. If the connection accidentally becomes loose it will disconnect by itself if the energy storage element is selected for providing enough force, so that an connection that has become untight by accident is released. It is preferred that the cannula cover is held slidably within the cannula holder for a sliding translation movement in longitudinal direction of the cannula. This allows a very compact connection and the cannula cover does not have to have a size taking up the collar of the reservoir.

In an alternative embodiment to the energy storing element the cannula cover is held within the cannula holder by guiding means imparting a rotational screw-in and screw-out movement to said cannula cover within the cannula holder during the insertion and the removing of the cannula holder from the receiving element.

In a preferred embodiment the locking means comprise a catch on the cannula cover adapted to lock into a recess of the cannula holder and the releasing means comprise a rib within the receiving element adapted to release the catch from the recess. The catch may be a two-stepped catch comprising a first block-shaped step and a second step provided held by an elastically deformable tongue.

Preferably, the connection system comprises blocking means and unblocking means for blocking of the cannula holder after full insertion into the device and unblocking of the cannula holder for releasing the cannula holder from the device, and in particular comprises unblocking means provided for one-handed operation of the means. This allows an easy operation of the connection system. The receiving element is preferably part of a medical device and in particular part of an insulin pump. This obviates the need for a separate adapter and the medical device takes up the forces in connection with the pumping of the liquid which allows a simpler construction of the connection. It is further preferred that the cannula holder is essentially L-shaped and wherein the cannula is held essentially in a 90 degree angle to the line exiting the cannula holder. This allows a full integration of the connection system into the shape of the medical device and results in an advantageous exit of the fluid line. It is further preferred that the receiving element is mounted to the device such that it is svivable about an axis for opening a chamber adapted to receive the liquid reservoir. This allows as well for a good adaptation of the connection system to a portable fluid pump.

The invention will be better understood and objects other than those set forth above will become apparent when consideration is given to the following detailed description of preferred embodiments of the invention by way of the drawings, wherein
Figure 1 shows a first embodiment of the connecting system wherein the receiving element is an adapter to be fixed to a device;
Figure 2 shows a sectional view of the cannula holder of Figure 1 with the cannula cover in a first state covering the cannula;
Figure 3 shows the cannula holder of Figure 2 with the cannula cover in the second state exposing the cannula;
Figure 4 shows a sectional view of the cannula holder of Figures 1 to 3 ready to be inserted in the receiving element of the system;
Figure 5 shows a sectional view of the cannula holder inserted into and fixed to the receiving element and with pierced ampoule;
Figure 6 shows a sectional view similar to Figure 4 but rotated 90 degrees about the longitudinal axis of the cannula so that the locking and releasing means can be seen;
Figure 7 shows the sectional view of Figure 6 with the cannula holder inserted within the receiving element;
Figure 8 shows a perspective view of the cannula cover with parts of the locking means;
Figure 9 shows a perspective view of a part of the cannula holder with other parts of the locking means;
Figure 10 shows a sectional view of the cannula holder with the cannula cover and a part of the receiving element with the releasing means;
Figure 11 shows in sectional view a part of the cannula holder inserted into the receiving element;
Figure 12 shows a sectional view of a second embodiment wherein the receiving element forms part of the device itself;
Figure 13 shows the cannula holder and the device in separated state before loading of the ampoule into the device;
Figure 14 shows a first step of the opening of the device;
Figure 15 shows a second step of the opening of the device;
Figure 16 shows the insertion of the ampoule;
Figure 17 and Figure 18 show the closing of the device;
Figure 19 shows the cannula holder in perspective view in exploded and assembled form;
Figure 20 shows an exploded view for explaining the blocking means of the second embodiment;
Figure 21 shows the elements of Figure 20 in assembled form;
Figure 22 shows the unblocking of the cannula holder;
Figure 23 shows the blocking and unblocking means of a third embodiment in exploded view;
Figure 24 shows the blocking and unblocking means in a sectional view;
Figure 25 shows another embodiment of the connection system with a form locked rotational movement of the cannula cover in exploded view; and
Figure 26 shows the embodiment of Figure 25 in sectional view before inserting the cannula holder into the receiving element.

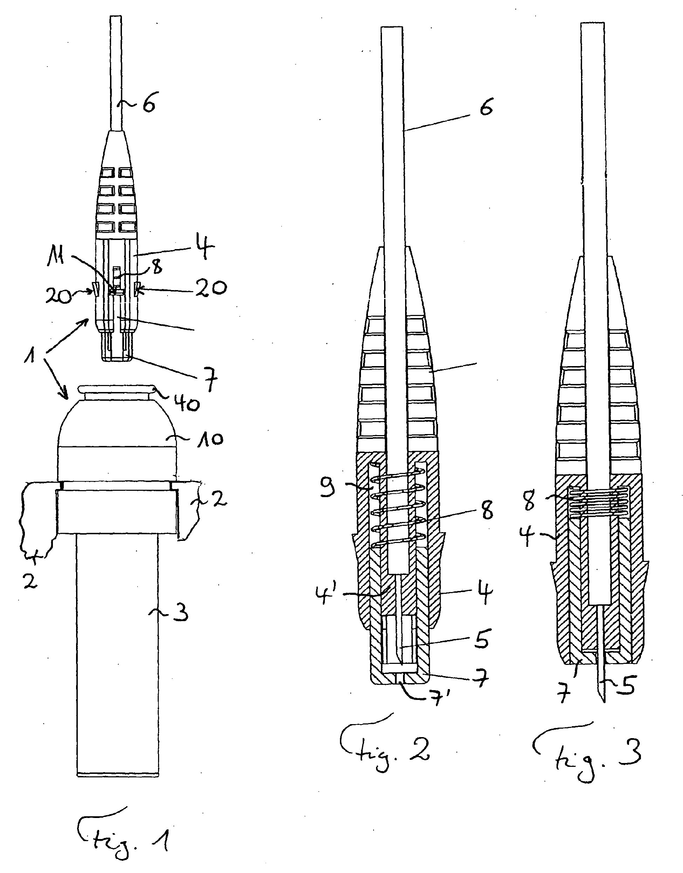

With reference to Figures 1 to 11 a first embodiment of the invention is now described. Figure 1 shows an overview of this first embodiment of the connecting system 1. There is provided a cannula holder 4 holding a cannula which can not be seen in Figure 1 since this cannula is covered by cannula cover 7 which prevents an accidental touching of the cannula by the user of the system. Within cannula holder 4 the cannula is in fluid connection with line 6 which shall be connected by the system to a reservoir 3 containing the fluid. This reservoir, in particular an ampoule, for example an ampoule containing insulin, is provided within a device 2 which is only indicated in this embodiment by a part of its housing. This device is for example a medical device and in particular an insulin pump for dosing insulin out of reservoir or ampoule 3, respectively, to a patient via line or tube 6 that leads to another cannula at its other end (not shown) for insertion into the body of the patient. The system according to this embodiment comprises a receiving element into which the cannula holder is to be inserted in the form of an adapter 10 which is has a shape so that it fits the device 2, and preferably surrounds the upper part of the reservoir 3. The cannula within the cannula holder 4 is inserted into the reservoir when the cannula holder is inserted into the receiving element, and thus in this embodiment into the adapter 10. Locking means generally shown as 11 prevent the cannula cover 7 to be moved or released, respectively, unless upon insertion of the cannula holder into the receiving element 10 whereupon releasing means within the receiving element 10 interact with the locking means 11 so that the cannula cover moves, and in particular slides backwards, away from the tip of the cannula, to expose the cannula within the receiving element 10, so that the reservoir 3 can be pierced by the cannula to establish the fluid connection between the reservoir and line 6. In case of an ampoule a septum closing the ampoule is pierced by the cannula. Blocking means, generally depicted as 20 in Figure 1, lock the fully inserted cannula holder 4 within the receiving element, and in this embodiment within the adapter 10. Unblocking means, part of which are generally shown as 40 in Figure 1, serve for releasing the cannula holder 4 from receiving element 10 and upon releasing the cannula holder the cannula cover is again brought in position as shown in Figure 1 to cover the cannula so that no contact of the person using the system with the cannula is possible, as will be explained in greater detail below. By this the person is protected against injury by the cannula on the one hand and on the other hand the cannula is protected against contamination.

The cannula holder of this embodiment is generally shown as having a cylindrical outer shape and consequently the cannula cover and the inner shape of the receiving element receiving the holder is shown cylindrically as well. Of course, other shapes can be selected as well. The cannula holder and the receiving element of the system will usually be made of plastic material and are preferably one-piece elements where shown in the drawings, but may of course as well be multi-piece elements.

Figures 2 and 3 show a sectional view of the cannula holder 4 of the first embodiment of the connection system 1. Same reference numerals as used before depict same elements or functionally equivalent elements, and are used for this embodiment and the further embodiments. In these sectional views the cannula 5 can be seen which is fixedly mounted within the cannula holder 4, for example by being fully enclosed by plastic core 4' of this cannula holder. Within holder 4 the cannula is in fluid connection with line 6, so that fluid entering the cannula at its tip enters the line 6. The cannula cover 7 fully covers cannula 5 in the first position or state, respectively, shown in Figure 2. The cover 7 is locked in this position by the locking means, which are not visible in this section, until the releasing means of the receiving element 10 (Figure 1) release the lock, as will be explained below. When the lock is released, the cover 7 can be moved, and in particular can slide backwards within space 9 in the holder 4, so that the cannula becomes exposed as can be seen in Figure 3, which shows the cover pushed fully backwards; because of the locking means, this second position or state, respectively, shown here in Figure 3 for explanation purposes for the "free" cannula holder 4 can only be assumed when this holder is inserted within the receiving element of the connection system 1. The cannula 5 passes through the small opening 7' of the cannula cover 7 during the backwards movement of the cannula cover. Preferably, an energy storage element, and in this embodiment a coil spring 8, is provided and stores energy that becomes available by the insertion force applied by the user of the system upon inserting the cannula holder 4 into the receiving element 10, so that this stored energy is available later when the cannula holder 4 is again removed from the receiving element 10 to push the cannula cover 7 back into the first position covering the cannula as shown in Figures 1 and 2. Then the locking means will again lock cover 7 in this position. The force exerted by coil spring 8 on the cannula cover 7 may be selected as being low by a corresponding spring size and spring rate, so that this force serves essentially only to push the cannula cover 7 back over the cannula 5 when the cannula holder has been unblocked by the user and is pulled out of the receiving element 10. On the other hand, and preferably, the spring force is selected as being so great that the spring 8 acting on the cannula cover 7 significantly helps pushing the cannula holder 4 out of the receiving element 10 by the cannula cover abutting on the receiving element 10 when the unblocking means have been actuated by the user.

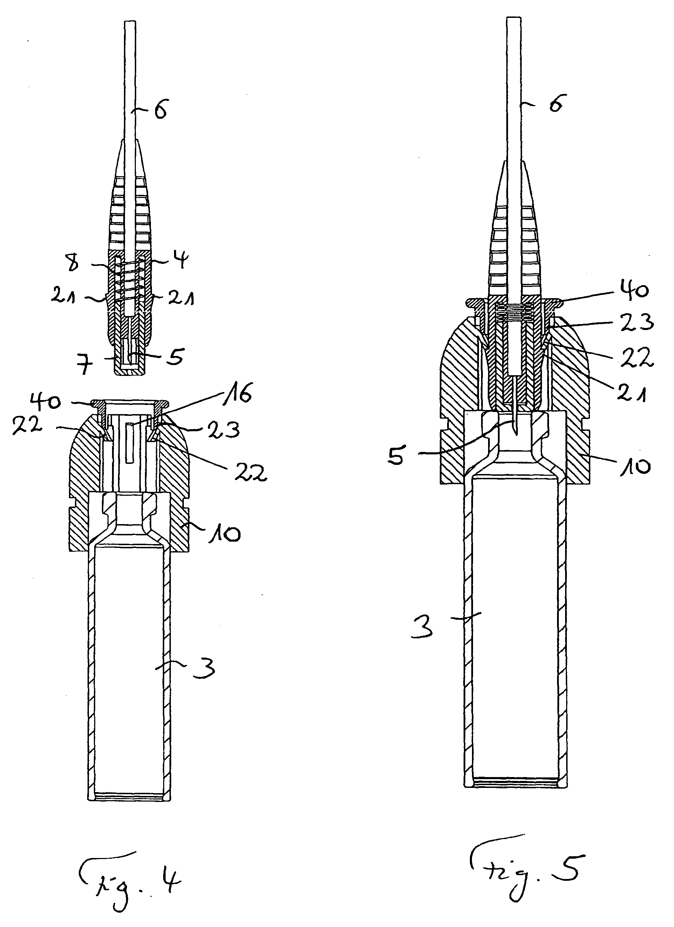

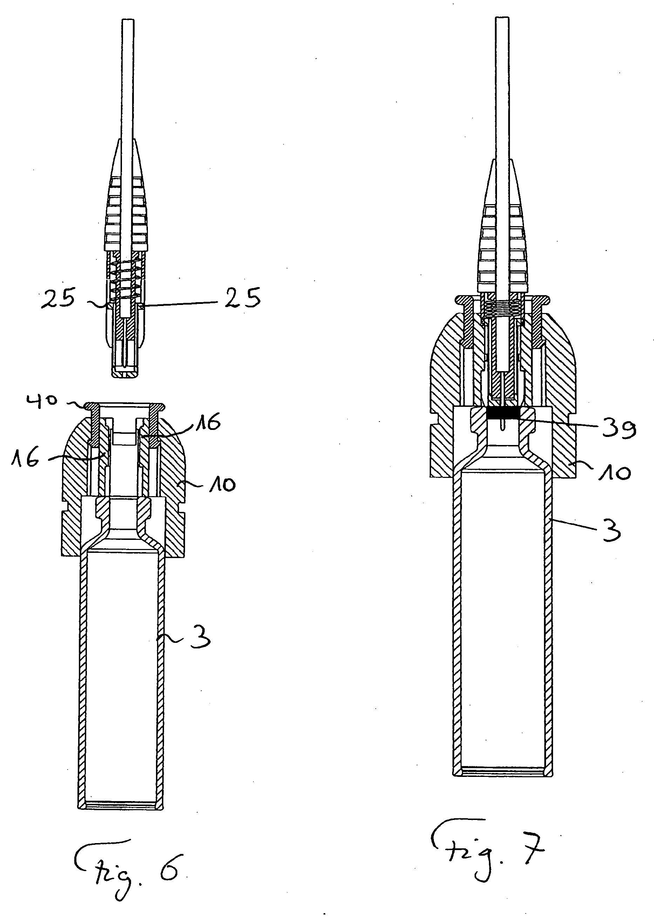

Figures 4 and 5 show the insertion of cannula holder 4 into the receiving element 10, which is in this embodiment the adapter within device 2, in sectional view. In the sectional view of Figure 4 a part of the releasing means within receiving element 10 can be seen in the form of an elongated protruding member or rib 16 that interacts with the locking means for the cannula cover 7 of cannula holder 4 when the holder 4 is inserted into the receiving element 10. In the section of Figures 4 and 5 the locking means (so far generally shown as means 11 in Figure 1) can not be seen, but will be shown and explained below with reference to Figures 6 to 11. On the one hand Figures 4 and 5 show the insertion of the cannula holder 4 and the piercing of the reservoir 3, in this case the ampoule 3, by the cannula 5 in the inserted position (Figure 5). On the other hand these Figures show the blocking means or elements, respectively, for securing the inserted holder 4 within the receiving element 10 and the unblocking means or elements, respectively, for the release of the holder 4 from the receiving element 10 by the user, which is usually done when an empty ampoule 3 and/or the line 6 with the cannulae on both ends have to be replaced. In this embodiment, the blocking elements are provided by interacting protrusions on the outside of the cannula holder 4 and on the inside of the receiving element 10. In particular, wedge shaped protrusions 21 are provided on the outer circumference of the holder 4 and corresponding latches 22 are provided on the inner circumference of the receiving element 10. The interaction of the protrusions and latches allows to insert the cannula holder 4 into the receiving element 10 and to block the holder in the fully inserted state. In the shown example the latches 22 are bended by the wedge shaped protrusions 21 during the insertion and spring back (into their normal unbent positions shown in Figures 4 and 5) in the fully inserted state of the cannula holder 4 and then contact the horizontal planes of the wedges, thus blocking the holder 4 in the fully inserted state. Several such interacting protrusions and latches are arranged around the holder 4 and the receiving element and the holder 4 and the receiving element 10 are keyed, for example by corresponding guiding means, so that the insertion can only be done in a position where the interacting protrusions align. This guiding is preferably provided by the interacting locking and releasing means for the cannula cover, but other matching keying elements can be provided to allow an insertion in a correct position only, so that interaction of the blocking elements occurs. Figures 4 and 5 show as well an unblocking element which is in this example a collar 40 that is slidably secured to the receiving element 10 and is provided with a ring 23, or with several fingers at the positions of the latches, that bend the latches 22 in radial direction away from the wedges 21 when the collar 40 is moved by finger pressure by the user in direction towards the cannula tip, so that the wedges are no longer blocked by the latches and thus cannula holder 4 can be pulled out of the receiving element 10 by the user. The pulling out is helped more or less, as outlined above, by the force of coil spring 8 that at the same time presses cannula cover 7, abutting on ampoule 3, over the cannula during the pull-out movement of the cannula holder.

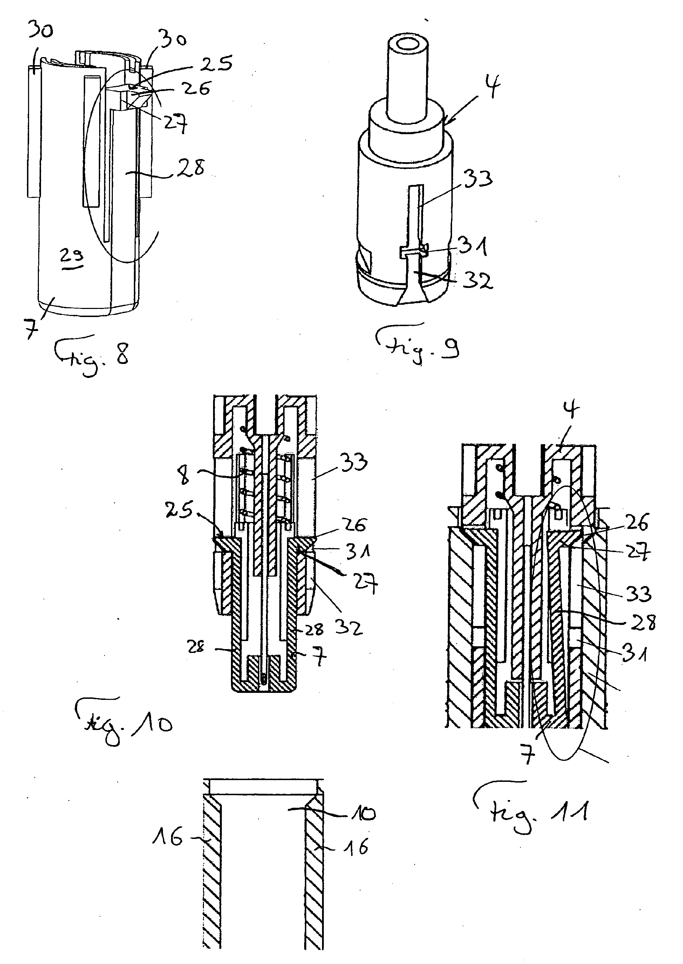

With reference to Figures 6 to 11 locking and releasing means for the cannula cover can now be described in greater detail. Figures 6, 7, 10 and 11 show sectional views wherein these means can be seen and Figures 8 and 9 show perspective views of the cannula cover 7 and the main part of the cannula holder 4 for a better explanation. Further reference is made to Figure 4 wherein a part of the releasing means is shown by rib 16 already mentioned. Cannula cover 7 is provided with a two step catch 25 having a narrower, wedge shaped step 26 and a wider, block shaped step 27, and preferably two such catches 25 are arranged opposed each other along the circumference of the cover 7. This catch, or each catch in the case of two catches or in the case of even more than two catches provided around the circumference of the holder, is formed at the end of an elastic tongue 28 which allows the catch to be bent in inward direction of the cannula cover, as can be seen in particular in Figure 11, and to spring back into its rest position shown in Figures 6, 8 and 10. This tongue 28 and the catch 25 is preferably in one piece with the wall 29 of the cannula cover 7 by providing slits into the wall which define the tongue. Further protruding ribs 30 may be provided on the outside of this wall 29 to provide sliding guides for the cannula cover within the holder 4 that is provided in this case with corresponding groves or slits taking up these sliding guides. The cannula holder 4 is provided with a recess 31 and particular a hole 31 for taking up the block shaped step 27 of catch 25 (and in the case of several catches/tongues is provided with the same number of corresponding holes 31 for the block shaped steps of these catches). When the cannula cover 7 is mounted in the cannula holder 4 and the cover 7 is in the first locked state covering the cannula, then block shaped step 27 of catch 25 sits in the correspondingly shaped hole 31 and the cannula cover is thus locked against movement in this position, for example shown in Figures 1 and 6. When the cannula holder 4 is inserted into the receiving element, in this case the adapter 10, then the releasing means will remove the catch from this locked position, allowing the moving of the cannula cover during the insertion of the cannula holder into the receiving element. In the shown example the rib 16 (and in the case of several catches the correspondingly placed several ribs 16) on the inside of the receiving element 10 first slides within a groove 32 of the cannula holder 4 leading to the hole 31. Then, when the insertion of the cannula cover into the receiving element 10 proceeds, rib 16 contacts the wedge shaped, narrower step 26 of catch 25 that protrudes into groove 32 (Figure 10). Rib 16 then forces catch 25 inwardly (in direction towards the cannula), thereby elastically deforming tongue 28. By this inwardly forcing the block shaped step 27 frees from hole 32 and the cover 7 can slide upwards (away from the cannula tip). During the upward movement step 26 of catch 25 is sliding within longitudinal slit 33 of the cannula holder 4 that connects to hole 31. When the cannula cover 7 is fully moved backwards (which as well corresponds with full insertion and the blocking of the cannula holder in the receiving element by the blocking means explained above) the position of Figure 11 is taken wherein the inwardly bent tongue 28 (bending shown only for the right tongue in the Figure) and step 26 and rib 16 can be seen. When the blocking means are unblocked and the cannula holder is pulled out of receiving element 10, rib 16 moves accordingly downwards (in direction towards the tip of the cannula) and the cannula cover 7 is pressed downwardly by coil spring 8 as well. During this downward movement catch 25 again reaches hole 31 where the block shaped step 27 of catch 25 again enters the hole due to the spring back elasticity of the tongue 28. Thus cover 7 moves during the pull out movement over the cannula again, covering same more and more, and is again locked in the state of fully covering the cannula when the cannula holder 4 is removed from the receiving element 10. Thus a fool prove cannula cover results, having a fully automatic function during insertion of the cannula holder into the receiving element and during pulling out of the cannula holder from the receiving element or adapter 10, respectively, and thus into and from device 2. In the fully inserted state shown in Figures 5 and 7 the cannula has pierced the reservoir 3 (and in case of an ampoule the septum 39 thereof) so that a fluid connection between reservoir 3 and line 6 is provided by means of the cannula.

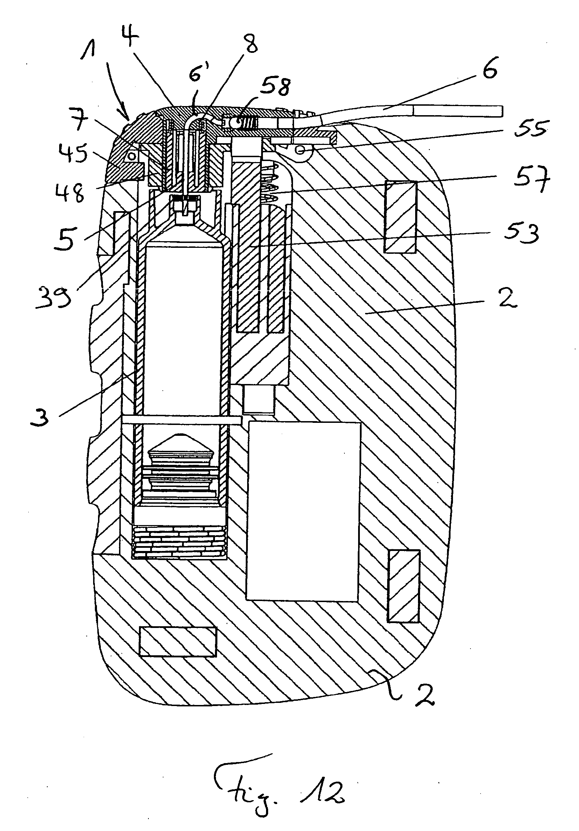

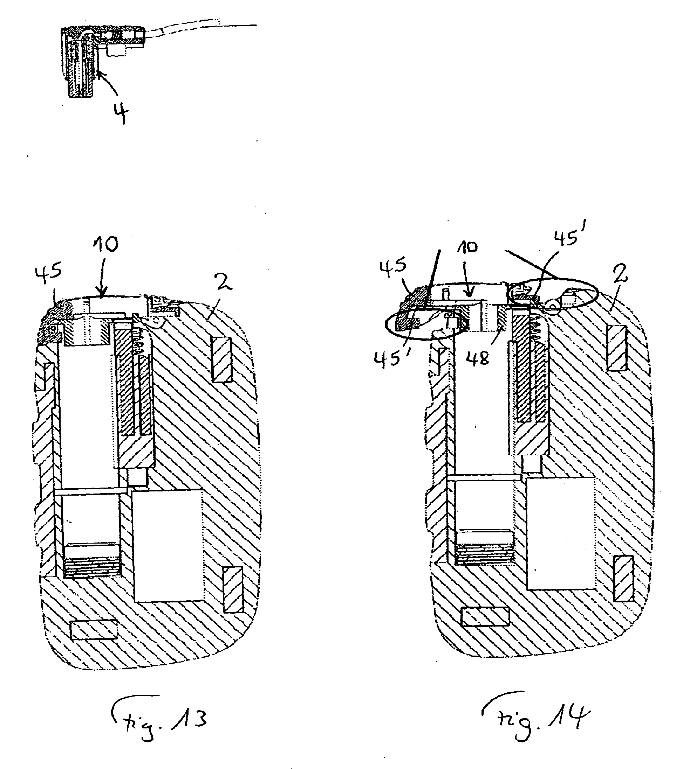

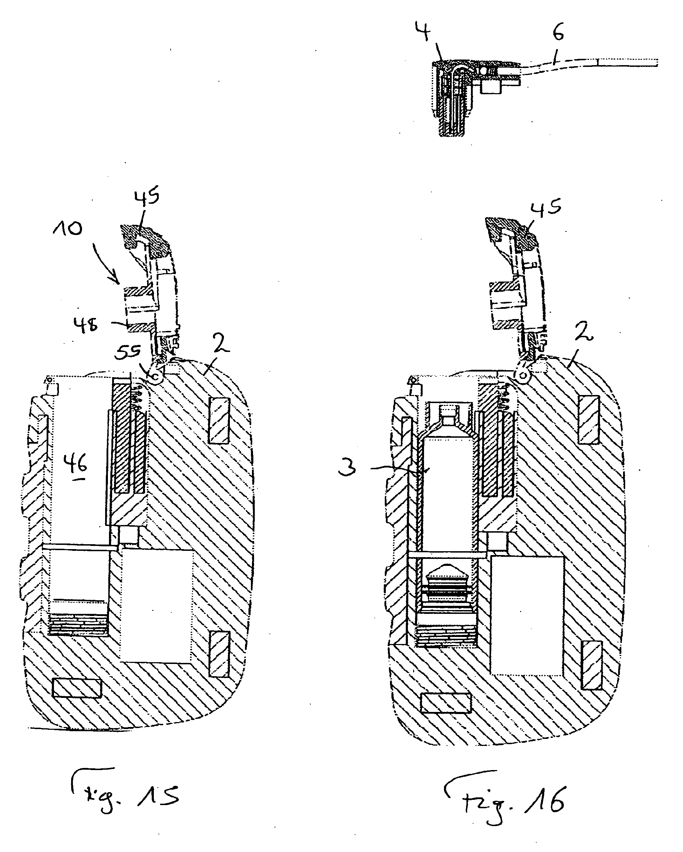

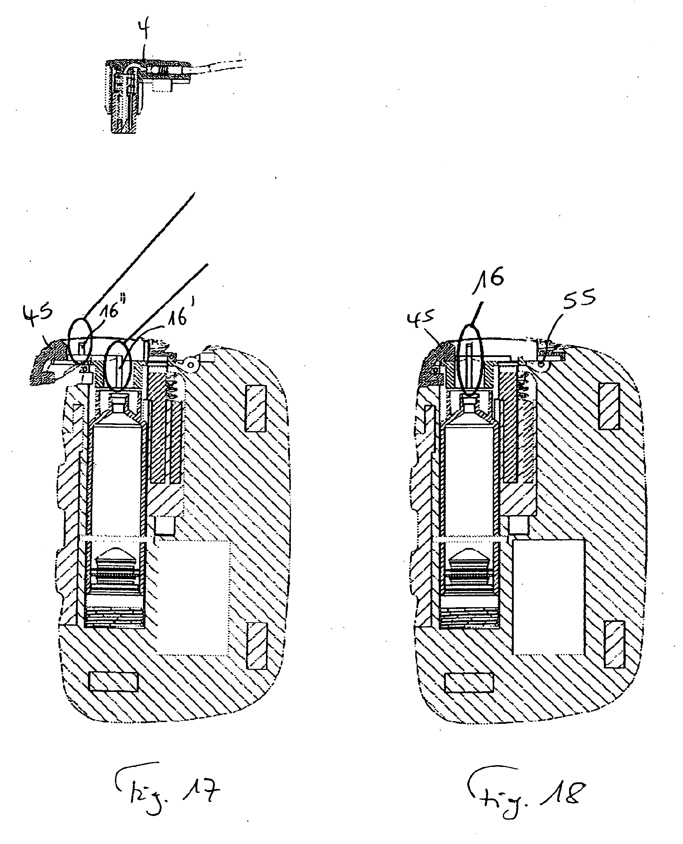

With reference to Figures 12 to 21 another embodiment of the invention is now described. Same reference numerals as before depict same elements or functionally equivalent elements. In this embodiment connection system 1 comprises a receiving element 10 that is not an adapter but forms part of the device 2 itself. Figure 12 shows a sectional view through a device 2 which is a portable medical pump, and for example an insulin pump, but of which only the functional elements relevant for the connection system are shown. Within this pump 2 a reservoir in form of an ampoule 3 is placed which is in fluid connection with line 6', 6 by means of cannula 5 having pierced the septum of the ampoule while the cannula holder 4 has been inserted into the receiving element 10 being part of device 2. The building elements of this embodiment are now explained in greater detail. Figure 13 shows the "empty" device 2 without an ampoule 3 and the cannula holder 4 pulled out of the device. The cannula holder 4 has essentially an L-shape wherein the cannula stands in a 90 degree angle to the line 6 as its exits the holder 4. The straight cannula 5 is connected to the line 6 for example via a curved conduit 6' and a valve 58 may be present between the conduit and line 6, allowing a fluid flow only by pressure of the pump. The part of the holder 4 where the cannula is held and the cannula cover 7 are still essentially cylindrical parts but may be of different shape as well. As shown in Figure 14 the receiving element 10 comprises for example a sliding lid 45, being slidable with regard to the part 48 for taking up the cannula holder, and comprising catch mechanisms 45' holding the sliding lid shut and allowing the opening by the user; the design of such mechanisms 45' for a lid is well known to person in the art and is not explained in detail here. The preferred receiving element 10 of this embodiment is further provided with an axis 55 that, after sliding the lid 45 to its opening position allows a swiveling motion of the receiving element 10 (Figure 15) to open the reservoir chamber 46 of the device 2 into which the ampoule can be inserted (Figure 16). The receiving element 10 is then swiveled back (Figure 17) and the lid is slid closed, so that the ampoule 3 is enclosed in the device (Figure 18). In this embodiment the releasing means uses at least one rib 16 as well, but this rib is provided by two rib sections 16' and 16" on the ampoule seat 48 and the sliding lid 45, respectively. These two rib sections 16' and 16" align to rib 16 when the lid is closed (Figure 18). The lid is held in its closed state for example by snapping means as catch mechanisms 45'. Any kind of closing means can be used that allow an easy closing and of course opening of the reservoir chamber by the pump user. At the same time the receiving element 10 and the closing means on this element must obviously be designed to hold the ampoule 3 firmly in place while the pump is pressing liquid out of the ampoule and - via cannula 5 - into line 6. The pump is for example working by a motor and spindle drive for a piston provided within the ampoule at the other end of the ampoule facing away from the cannula. This way of driving the fluid out of the ampoule is known in the medical pump art and is therefore not explained in detail here. The design of a cover for the ampoule chamber 46 that can be opened and closed and at the same time is providing the second part 10 of the connection system for inserting the cannula holder and holding the ampoule in place can chosen in different ways by the man skilled in the art; the sliding lid and swivable element 10 as explained so far and shown in the drawings is a preferred example only.

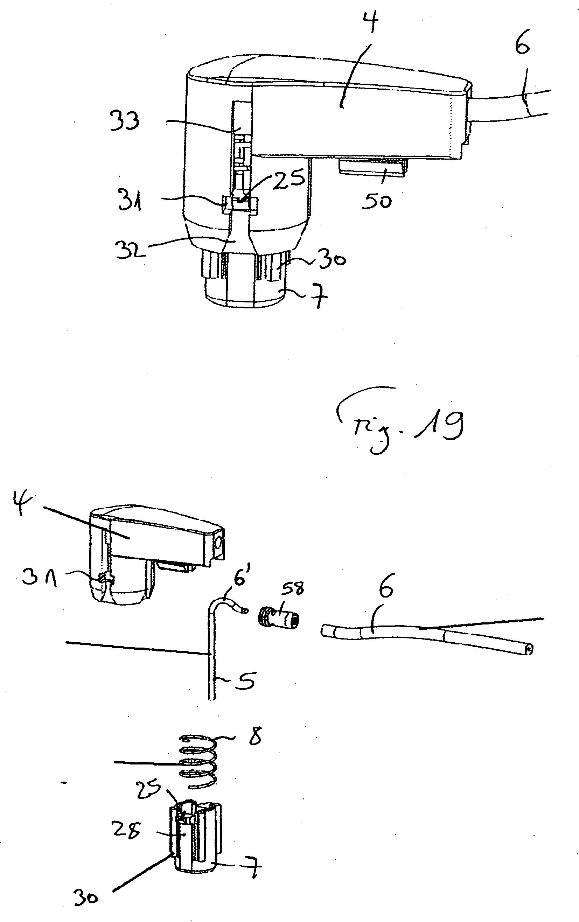

Now, referring again to Figure 18, the rib 16 as preferred example for the releasing means (and preferably at least two of such ribs facing each other on the inner circumference of the cylindrical seat 48 for the cannula holder) is ready to act on the locking means for the cannula cover 7 of cannula holder 4. The locking means are preferably means constructed identically as explained in the former embodiment of Figures 1 to 11 and thus Figure 19 of the second embodiment (showing an exploded view and an assembled view of cannula holder 4 in the same Figure) shows the cannula holder with the twostepped catch 25 within hole 31 as well and with groove 32 for the rib and slit 33 for the second, narrower step 26. The function of the locking and releasing means is therefore identical with the first embodiment. Cannula cover 7 is provided with the two step catch 25 having a narrower, wedge shaped step 26 and a wider, block shaped step 27, and preferably two such catches are arranged opposed each other along the circumference of the cover 7. This catch, or each catch in the case of two catches or in the case of even more than two catches provided around the circumference of the holder, is formed at the end of an elastic tongue 28 which allows the catch to be bent in inward direction of the cannula cover, as seen in Figure 11 and to spring back into its rest position shown in Figures 6, 8 and 10. This tongue 28 and the catch 25 can be in one piece with the wall 29 of the cannula cover 7. Further protruding ribs 30 may be provided on the outside of this wall 29 to provide sliding guides for the cannula cover within the holder 4 that is provided in this case with corresponding groves or slits taking up these sliding guides. The cannula holder 4 is provided with the hole 31 for taking up the block shaped step 27 of catch 25 (and in the case of several catches/tongues is provided with the same number of corresponding holes 31 for the block shaped steps of these catches). When the cannula cover 7 is mounted in the cannula holder 4 and the cover 7 is in the locked state covering the cannula, then catch block shaped step 27 of catch 25 fits in the correspondingly shaped hole 31 and the cannula cover is thus locked against movement in this position, for example shown in Figures 1 and 6. When the cannula holder 4 is inserted into the receiving element 10, then the releasing means will remove the catch from this locked position, allowing the moving of the cannula cover during the insertion movement of the cannula holder into the receiving element. In the shown example the rib 16 (and in the case of several catches the correspondingly placed several ribs 16) on the inside of the receiving element 10 first slides within a groove 32 of the cannula holder 4 leading to the hole 31. Then, when the insertion of the cannula cover into the receiving element 10 proceeds, rib 16 contacts the wedge shaped, narrower step 26 of catch 25 that protrudes into groove 32 (as explained before with reference to Figure 10). The rib 16 then forces catch 25 inwardly (in direction towards the cannula), thereby elastically deforming tongue 28. By this inwardly forcing block shaped step 27 frees from hole 32 and the cover 7 can slide upwards (away from the cannula tip). During the upward movement step 26 of catch 25 is sliding within slit 33 of the cannula holder 4 that connects to hole 31. When the cannula cover 7 is fully moved backwards (which as well corresponds with the blocking of the cannula cover in the receiving element by the blocking means explained above) the position of Figure 11 is taken wherein the inwardly bent tongue 28 and step 26 and rib 16 can be seen. When the blocking means are unblocked and the cannula holder is pulled out of receiving element 10, rib 16 moves accordingly downwards (in direction towards the tip of the cannula) and the cannula cover is pressed downwardly by coil spring 8 as well. During this downward movement catch 25 again reaches hole 31 where the block shaped step 27 of catch 25 again enters the hole due to the spring back elasticity of the tongue 28. Thus cover 7 has moved during the pull out movement over the cannula covering same more and more and is again locked in the state of fully covering the cannula when the cannula holder 4 is fully removed from the receiving element 10 and thus from device 2. Thus a fool-prove cannula cover results with fully automatic function during insertion of the cannula holder into and pulling out of the cannula holder from the receiving element and thus from device 2. In the fully inserted state shown in Figure 12 the cannula has pierced the reservoir 3 (and in case of an ampoule the septum 39 thereof) so that a fluid connection between reservoir 3 and line 6 is provided by means of the cannula.

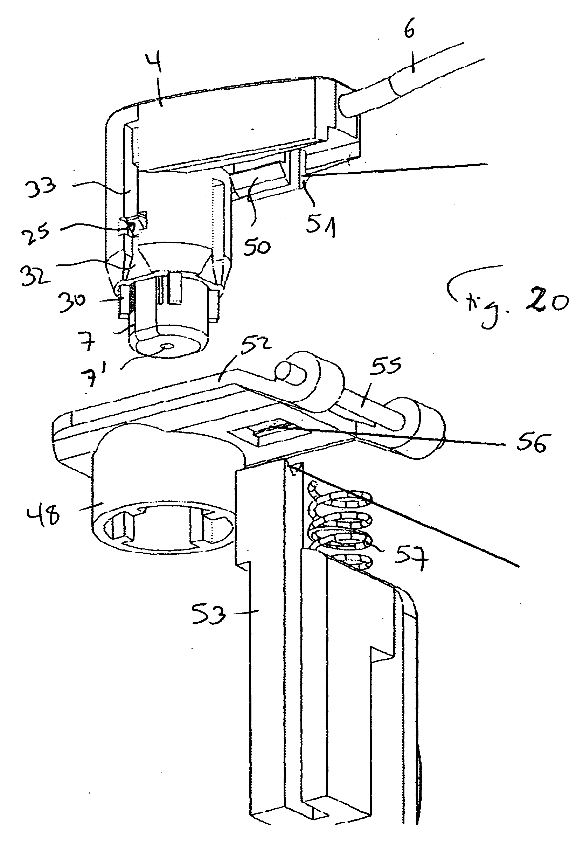

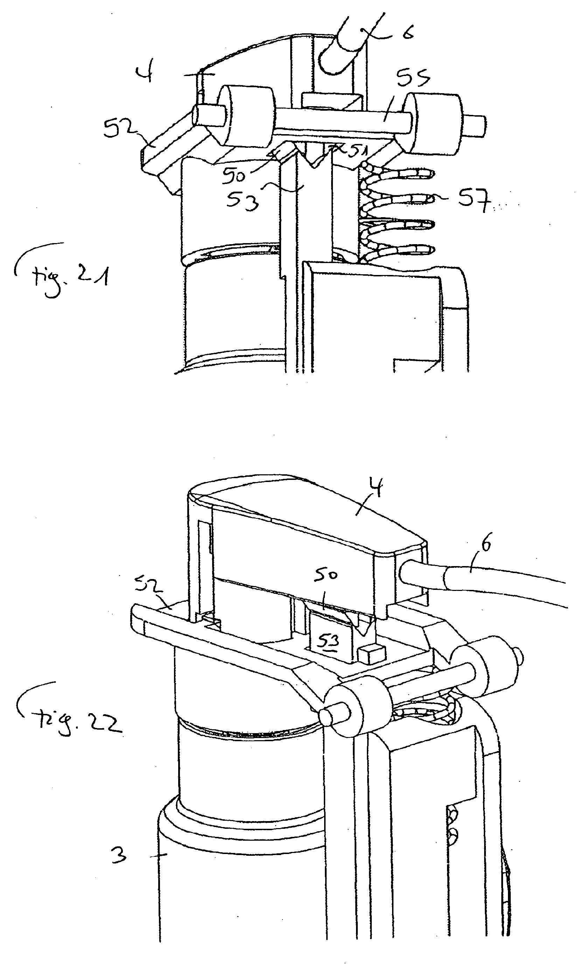

The blocking and unblocking means of this embodiment could have been selected identical to the blocking and unblocking means of the first embodiment but have been selected differently from the first embodiment. Here two latches 50 and 51 are provided on the lower outside of the horizontal part of the L-shaped cannula holder as can be seen in Figures 19 and 20. When the cannula holder is inserted into the receiving element 10 these latches snap into a hole 56 within plate 52 of the cover when the cannula holder 4 is fully inserted into device 2, as can be seen in Figure 21. To unblock the cannula holder the two latches 50 and 51 are pressed together by a V-shaped lifter 53 that can be manually actuated by a knob on the outside of the pump housing against the force of spring 57 so that the latches come free of plate 52 and are pressed upwardly and cannula holder 4 is unblocked. Figure 22 shows the action of the lifter 53 that has already pushed the latches 50 and 51 away from the plate 52.

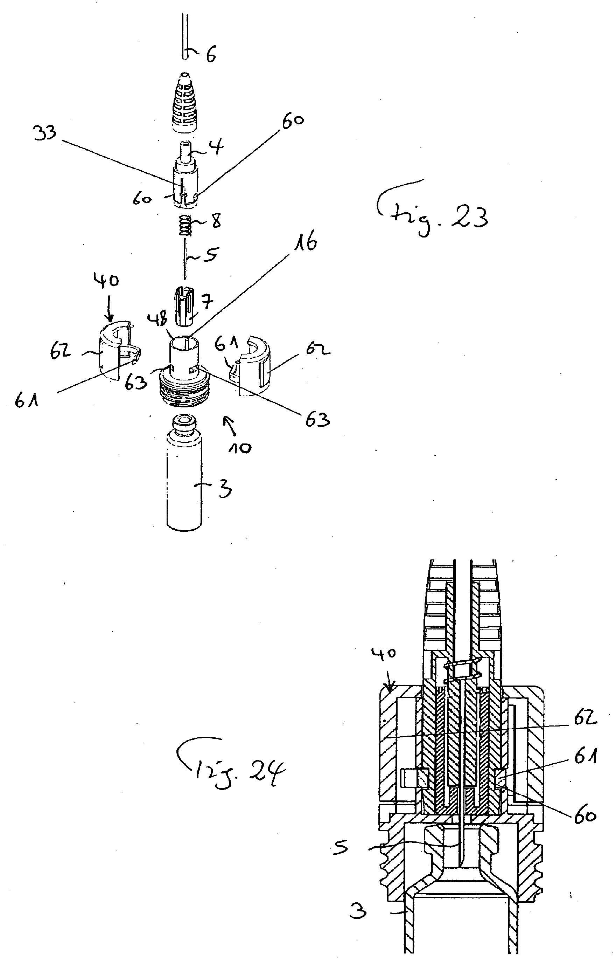

With reference to Figures 23 and 24 a further embodiment is described with different means for blocking and unblocking of the cannula holder. Figure 23 shows an exploded view of the cannula holder 4 and the receiving element 10 which is here again of the adapter type. The cannula holder and the locking means and the releasing means for the cannula cover are of the same construction as described for the first embodiment and reference is made to this description above. The blocking means and the unblocking means on the other hand comprise in this embodiment at least one recess 60 in the outer wall of cannula holder 4 and at least one corresponding finger 61 on the adapter 10 arranged to reach into the recess 60 when the cannula holder is fully inserted into adapter 10, so that the cannula holder is blocked within adapter 10 by the finger. The embodiment shown in Figures 23 and 24 comprises two recesses 60 and two corresponding fingers. The fingers 61 reach so far through slit-shaped holes 63 in the wall of seat 48 of adapter 10 so that they can enter the recesses 60. On the other hand the fingers are connected to knobs 62 that can be pressed together by the user. By this pressure the fingers 61 make a movement away from the cannula holder 4 and its recesses and the cannula holder is unblocked and will be pushed out of seat 48 and thus out of adapter 10 by the force of spring 8.

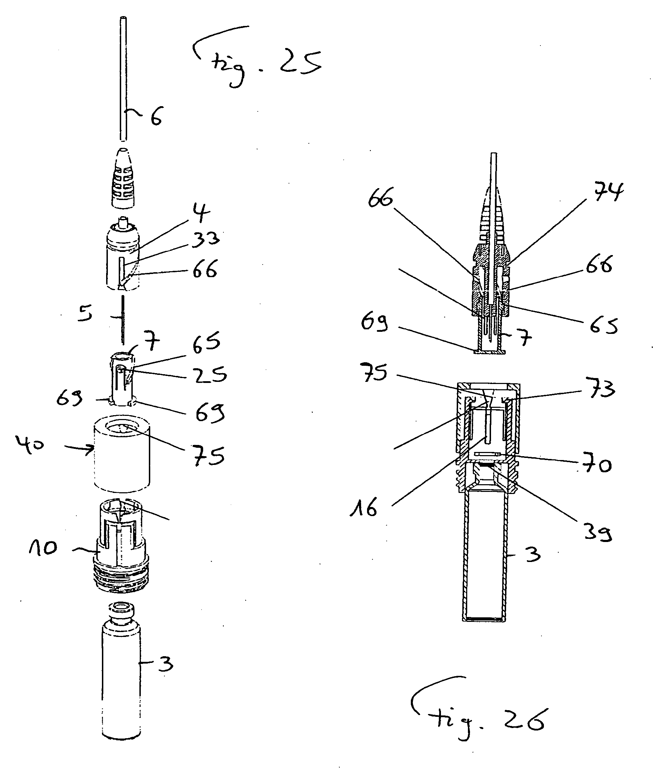

Figures 25 and 26 show another embodiment wherein no energy storage element 8 is provided. In this embodiment the cannula cover 7 is provided with a screw thread shaped protrusion 65 that is guided in a corresponding screw thread shaped guiding slit 66 in the wall of the cannula holder 4. Thus the cannula cover 7 will be move backwards in a rotating movement when the locking means here provided by at least one catch 67 (similar to the catch 25 of the other embodiments) are unlocked by the releasing means which are provided here as well by a rib or ribs 16. The rib or ribs 16 prevent on the other hand a turning of the cannula holder with the guiding slit, so that only the cannula cover pushed backwards rotates by abutting either on the adapter ground 68 or on the ampoule 3 while the cannula holder 4 is inserted into the adapter 10 and the screw thread shaped guiding means 65, 66 force the rotating movement. The cannula is exposed by the rotating backwards movement of the cannula cover 7. As well by the rotating movement of the cannula cover 7 noses 69 on this cover rotate and enter by this turning movement below protrusions 71 on the inside wall of seat 48 of the adapter 10. The cannula cover 7 is therefore held within adapter 10 against pulling out from the adapter and thus, when the cannula holder 4 is again pulled out from the adapter 10, the cannula cover 7 is pulled over the cannula 5 again by making the rotating movement by the guiding means 65, 66 in opposite direction compared to the insertion. By this rotating movement the cannula cover 7 is finally free again from protrusions 71 since noses 70 turn free from these protrusions by the rotating movement as well, so that the cannula holder 4 with the cover 7 can be pulled out of the adapter 10. The blocking and unblocking means of this embodiment can be similar to the corresponding means of the first embodiment. Different blocking and unblocking means are shown in Figures 25 and 26. Catches 73 are provided on the adapter 10 that are arranged to fit into recesses 74 on the outside of the cannula holder 4 when this holder is fully inserted into the adapter 10. A wedge 75 on the unblocking means is adapted to force the catches 73, which are correspondingly wedge-shaped, out of the recesses 74 when the cannula holder 4 shall be unblocked from the receiving element 10, as has generally be explained before. The details of such an blocking and unblocking are not shown for this embodiment, since the detail construction of such means can easily be conceived by the skilled person.

## Claims

1. A connection system (1) for connecting a fluid line (6) to a fluid reservoir (3) located within a device (2) by way of a cannula (5), comprising
- a cannula holder (4) holding the cannula (5) and comprising a movable cannula cover (7) and locking means (11) for said cannula cover (7), preventing the movement of the cannula cover (7) by locking said cannula cover in a first state covering the cannula,
- a receiving element (10) for receiving the cannula holder (4), the receiving element being adapted for fixation to the device (2) or the receiving element forming part of the device (2), said receiving element comprising releasing means (16) adapted for interaction with the locking means (11), and wherein
- the releasing means (16) and the locking means (11) are interactingly arranged to release the cannula cover (7) by said releasing means interacting with the locking means, so that the cannula cover is movable by the inserting force to expose the cannula when the cannula holder (4) is inserted into the receiving element (10), so that the cannula cover (7) is in a second state exposing the cannula when the cannula holder (4) is inserted within the receiving element (10).

2. A connection system according to claim 1 wherein the cannula cover (7) is moved by a energy storage means (8), and in particular a coil spring (8), in opposite direction compared to the movement during insertion, while the cannula holder (4) is removed from the receiving element.

3. A connection system according to claim 1 or 2 wherein the cannula cover (7) is held slidably within the cannula holder (4) for a sliding translation movement in longitudinal direction of the cannula (5).

4. A connection system according to claim 1 wherein the cannula cover (7) is held within the cannula holder (4) by guiding means (65, 66) imparting a rotational screw-in and screw-out movement to said cannula cover (7) within the cannula holder (4) during the insertion and the removing of the cannula holder from the receiving element.

5. A connection system according to any of claims 1 to 4 wherein the locking means comprise a catch (25) on the cannula cover (7) adapted to lock into a recess (31) of the cannula holder and the releasing means (16) comprise a rib within the receiving element (10) adapted to release the catch (25) from the recess.

6. A connection system according to any of claims 1 to 5 wherein the catch is a two-stepped catch comprising a first block-shaped step (27) and a second step (26) provided held by an elastically deformable tongue (28).

7. A connection system according to any of the preceding claims comprising blocking means (20) and unblocking means (40) for releasably blocking the cannula holder (4) within the receiving element (10) and in particular comprising unblocking means provided for one-handed operation of the means.

8. A connection system according to any of the preceding claims wherein the receiving element (10) is part of a medical device (2) and in particular part of an insulin pump.

9. A connection system according to any of the preceding claims wherein the cannula holder (4) is essentially L-shaped and wherein the cannula (5) is held essentially in a 90 degree angle to the line (6) exiting the cannula holder.

10. A connection system according to claim 8 or 9 wherein the receiving element (10) is mounted to the device (2) such that it is svivable about an axis (55) for opening a chamber (46) adapted to receive the liquid reservoir (3).

11. A connection system according to the preceding claims wherein the device is a medical pumping device.

12. A connection system according to claim 11 wherein the medical pumping device is a portable medication pump and in particular an insulin pump.

## Patentansprüche

1. Verbindungssystem (1) zum Verbinden einer Flüssigkeitsleitung (6) mit einem Flüssigkeitsreservoir (3), das sich in einer Vorrichtung (2) befindet, mittels einer Kanüle (5), umfassend:
- eine Kanülenhalterung (4), welche die Kanüle (5) hält und eine bewegbare Kanülenabdeckung (7) und Arretiermittel (11) für die genannte Kanülenabdeckung (7) umfasst, welche die Bewegung der Kanülenabdeckung (7) durch Arretieren der genannten Kanülenabdeckung in einem ersten Zustand, in dem die Kanüle abgedeckt ist, verhindern,
- ein Aufnahmeelement (10) zum Aufnehmen der Kanülenhalterung (4), wobei das Aufnahmeelement zur Fixierung an der Vorrichtung (2) eingerichtet ist oder das Aufnahmeelement einen Teil der Vorrichtung (2) bildet, wobei das genannte Aufnahmeelement Freigabemittel (16) umfasst, die zum Zusammenwirken mit den Arretiermitteln (11) eingerichtet sind, und wobei
- die Freigabemittel (16) und die Arretiermittel (11) zusammenwirkend angeordnet sind, um die Kanülenabdeckung (7) freizugeben, indem die genannten Freigabemittel mit den Arretiermitteln zusammenwirken, so dass die Kanülenabdeckung durch die Einführungskraft bewegbar ist, um die Kanüle freizulegen, wenn die Kanülenhalterung (4) in das Aufnahmeelement (10) eingeführt wird, so dass die Kanülenabdeckung (7) sich in einem zweiten Zustand befindet, in dem die Kanüle freigelegt ist, wenn die Kanülenhalterung (4) in das Aufnahmeelement (10) eingeführt ist.

2. Verbindungssystem nach Anspruch 1, wobei die Kanülenabdeckung (7) durch ein Energiespeichermittel (8) und insbesondere eine Spiralfeder (8) in die entgegengesetzte Richtung im Vergleich zu der Bewegung während des Einführens bewegt wird, während die Kanülenhalterung (4) aus dem Aufnahmeelement entfernt wird.

3. Verbindungssystem nach Anspruch 1 oder 2, wobei die Kanülenabdeckung (7) für eine Verschiebungsübertragungsbewegung in Längsrichtung der Kanüle (5) verschiebbar in der Kanülenhalterung (4) gehalten wird.

4. Verbindungssystem nach Anspruch 1, wobei die Kanülenabdeckung (7) in der Kanülenhalterung (4) durch Führungsmittel (65, 66) gehalten wird, die während des Einführens und des Entfernens der Kanülenhalterung aus dem Aufnahmeelement eine Einschraub- und Ausschraubdrehbewegung auf die Kanülenabdeckung (7) in der Kanülenhalterung (4) vermitteln.

5. Verbindungssystem nach irgendeinem der Ansprüche 1 bis 4, wobei die Arretiermittel eine Klinke (25) auf der Kanülenabdeckung (7) umfassen, die dazu eingerichtet ist, in eine Ausnehmung (31) der Kanülenhalterung einzurasten, und die Freigabemittel (16) eine Rippe in dem Aufnahmeelement (10) umfassen, die dazu eingerichtet ist, die Klinke (25) aus der Ausnehmung zu lösen.

6. Verbindungssystem nach irgendeinem der Ansprüche 1 bis 5, wobei die Klinke eine zweistufige Klinke ist, die eine erste blockförmige Stufe (27) und eine zweite Stufe (26) umfasst, die von einer elastisch verformbaren Zunge (28) gehalten bereitgestellt sind.

7. Verbindungssystem nach irgendeinem der vorhergehenden Ansprüche, das Sperrmittel (20) und Entsperrmittel (40) zum lösbaren Sperren der Kanülenhalterung (4) in dem Aufnahmeelement (10) umfasst und insbesondere Entsperrmittel umfasst, die zum einhändigen Bedienen der Mittel vorgesehen sind.

8. Verbindungssystem nach irgendeinem der vorhergehenden Ansprüche, wobei das Aufnahmeelement (10) Teil einer medizinischen Vorrichtung (2) und insbesondere Teil einer Insulinpumpe ist.

9. Verbindungssystem nach irgendeinem der vorhergehenden Ansprüche, wobei die Kanülenhalterung (4) im Wesentlichen L-förmig ist und wobei die Kanüle (5) im Wesentlichen in einem 90-Grad-Winkel zu der Leitung (6) gehalten wird, die aus der Kanülenhalterung austritt.

10. Verbindungssystem nach Anspruch 8 oder 9, wobei das Aufnahmeelement (10) derart an der Vorrichtung (2) angebracht ist, dass es zum Öffnen einer Kammer (46), die zum Aufnehmen des Flüssigkeitsreservoirs (3) eingerichtet ist, um eine Achse (55) schwenkbar ist.

11. Verbindungssystem nach den vorhergehenden Ansprüchen, wobei die Vorrichtung eine medizinische Pumpvorrichtung ist.

12. Verbindungssystem nach Anspruch 11, wobei die medizinische Pumpvorrichtung eine tragbare Medikamentenpumpe und insbesondere eine Insulinpumpe ist.

## Revendications

1. Système de liaison (1) pour relier une conduite de fluide (6) à un réservoir de fluide (3) situé à l'intérieur d'un dispositif (2) au moyen d'une canule (5), comprenant
- un porte-canule (4) portant la canule (5) et comprenant un couvercle de canule mobile (7) et des moyens de verrouillage (11) pour ledit couvercle de canule (7), empêchant le mouvement du couvercle de canule (7) par verrouillage dudit couvercle de canule dans un premier état recouvrant la canule ;
- un élément de réception (10) pour recevoir le porte-canule (4), l'élément de réception étant apte à être fixé au dispositif (2) ou l'élément de réception faisant partie du dispositif (2), ledit élément de réception comprenant des moyens de libération (16) aptes à interagir avec les moyens de verrouillage (11), et dans lequel
- les moyens de libération (16) et les moyens de verrouillage (11) sont disposés de façon interactive pour libérer le couvercle de canule (7) par lesdits moyens de libération interagissant avec les moyens de verrouillage, de telle sorte que le couvercle de canule est déplaçable par la force d'introduction pour exposer la canule lorsque le porte-canule (4) est introduit dans l'élément de réception (10), de telle sorte que le couvercle de canule (7) est dans un second état exposant la canule lorsque le porte-canule (4) est introduit à l'intérieur de l'élément de réception (10).

2. Système de liaison selon la revendication 1, dans lequel le couvercle de canule (7) est déplacé par un moyen de stockage d'énergie (8), et en particulier un ressort hélicoïdal (8), en direction opposée par comparaison avec le mouvement pendant l'introduction, alors que le porte-canule (4) est retiré de l'élément de réception.

3. Système de liaison selon l'une des revendications 1 ou 2, dans lequel le couvercle de canule (7) est maintenu de façon coulissante à l'intérieur du porte-canule (4) en vue d'un mouvement de translation coulissant en direction longitudinale de la canule (5).

4. Système de liaison selon la revendication 1, dans lequel le couvercle du canule (7) est maintenu à l'intérieur du porte-canule (4) par des moyens de guidage (65, 66) conférant un mouvement tournant de vissage et de dévissage audit couvercle de canule (7) à l'intérieur du porte-canule (4) pendant l'introduction et le retrait du porte-canule à partir de l'élément de réception.

5. Système de liaison selon l'une quelconque des revendications 1 à 4, dans lequel les moyens de verrouillage comprennent un organe d'encliquetage (25) sur le couvercle de canule (7) apte à se verrouiller dans une cavité (31) du porte-canule et les moyens de libération (16) comprennent une nervure à l'intérieur de l'élément de réception (10) apte à libérer l'organe d'encliquetage (25) de la cavité,

6. Système de liaison selon l'une quelconque des revendications 1 à 5, dans lequel l'organe d'encliquetage est un organe d'encliquetage à deux étages comprenant un premier étage en forme de bloc (27) et un second étage (26), disposés maintenu par une languette élastiquement déformable (28).

7. Système de liaison selon l'une quelconque des revendications précédentes, comprenant des moyens de blocage (20) et des moyens de déblocage (40) pour bloquer de façon libérable le porte-canule (4) à l'intérieur de l'élément de réception (10) et en particulier comprenant des moyens de déblocage disposés pour un actionnement d'une main des moyens.

8. Système de liaison selon l'une quelconque des revendications précédentes, dans lequel l'élément de réception (10) fait partie d'un dispositif médical (2) et en particulier fait partie d'une pompe à insuline.

9. Système de liaison selon l'une quelconque des revendications précédentes, dans lequel le porte-canule (4) est essentiellement en forme de L et dans lequel la canule (5) est maintenue essentiellement dans un angle de 90° par rapport à la conduite (6) sortant du porte-canule.

10. Système de liaison selon l'une des revendications 8 ou 9, dans lequel l'élément de réception (10) est monté sur le dispositif (2) de telle sorte qu'il est apte à pivoter autour d'un axe (55) pour ouvrir une chambre (46) apte à recevoir le réservoir de liquide (3).

11. Système de liaison selon l'une des revendications précédentes, dans lequel le dispositif est un dispositif de pompage médical.

12. Système de liaison selon la revendication 11, dans lequel le dispositif du pompage médical est une pompe à médicament portable et en particulier une pompe à insuline.
